# EUROPEAN PATENT SPECIFICATION

(11) **EP 2 125 772 B1**
(45) Date of publication and mention of the grant of the patent: **11.09.2013**
(21) Application number: 07858827.4
(22) Date of filing: 20.12.2007
(51) Int. Cl.: C07D 333/20

(54) **A PROCESS FOR THE PREPARATION OF DULOXETIN AND NEW KEY INTERMEDIATES FOR USE THEREIN**
VERFAHREN ZUR HERSTELLUNG VON DULOXETIN UND NEUE SCHLÜSSELZWISCHENPRODUKTE, DIE DABEI VERWENDET WERDEN
PROCÉDÉ DE PRÉPARATION DE DULOXÉTINE ET NOUVEAUX INTERMÉDIAIRES CLÉS POUR UNE UTILISATION DANS CELUI-CI.

(30) Priority: 22.12.2006 HU 0600946
(43) Date of publication of application: 02.12.2009
(73) Proprietor: Richter Gedeon Nyrt., 1103 Budapest (HU)
(72) Inventor: BÓDI, József, H-1202 Budapest (HU); SZÖKE, Katalin, 1105 Budapest (HU); ÉLES, János, 1121 Budapest (HU); FOGASSY, Elemér, 2030 Érd (HU); SCHINDLER, József, 2151 Fót (HU); VUKICS, Krisztina, 1111 Budapest (HU); FARAGÓ, János, H-2730 Albertirsa (HU); TEMESVÁRI, Krisztina, 1157 Budapest (HU); GÁTI, Tamás, 1114 Budapest (HU)
(74) Representative: HOFFMANN EITLE
(86) International application number: PCT/HU2007/000127
(87) International publication number: WO 2008/078124

(56) References cited:
- EP-A- 1 506 965
- WO-A-2004/031168
- WO-A-2006/027798
- DATABASE CAPLUS CHEMICAL ABSTRACTS SERVICE, COLUMBUS, OHIO, US; XP002473217 retrieved from STN accession no. 2006:584790 & CN 1 687 057 A (SHANGDI XINSHIJI BIOLOG MEDICI [CN]) 26 October 2005 (2005-10-26)
- DEETER J ET AL: "ASYMMETRIC SYNTHESIS AND ABSOLUTE STEREOCHEMISTRY OF LY248686" TETRAHEDRON LETTERS, ELSEVIER, AMSTERDAM, NL, vol. 31, no. 49, 26 November 1990 (1990-11-26), pages 7101-7104, XP001119089 ISSN: 0040-4039 cited in the application

## Description

The present invention relates to a process for the preparation of duloxetin ((S)-(+)-N-methyl-3-(1-naphthalenyloxy)-3-(2-thienyl)propanamine) of formula (I) and pharmaceutically acceptable salts thereof comprising resolution of the racemic 3-(N-methyl-N-benzylamino)-1-(2-thienyl)-1-propanol of formula (II) by treatment with a derivative of D-phenylglycin of general formula (III) wherein
R¹, R² an R³ represent independently of each other hydrogen, halogen, cyano, nitro or amino optionally substituted by one or more C₁₋₄ alkyl group; trifluoromethyl, C₁₋₄ alkyl, C₁₋₄ alkoxy, -C(=O)-NH₂, C₁₋₄ alkoxycarbonyl, trifluoromethoxy or hidroxy to form (S)-3-(N-methyl-N-benzylamino)-1-(2-thienyl)-1-propanol of formula (IV) which is then reacted with 1-fluoronaphtalene resulting (S)-N-methyl-N-benzyl-3-(1-naphthalenyloxy)-3-(2-thienyl)propanamine of formula (V) which is debenzylated by (1-chloroethyl)-chloroformate.

Accordingly, one object of the invention is a process for the preparation of duloxetin ((S)-(+)-N-methyl-N-(3-(1-naphthalenyloxy)-3-(2-thienyl)propanainine)) of formula (I) and pharmaceutically acceptable salts thereof. This process is outlined as follows.

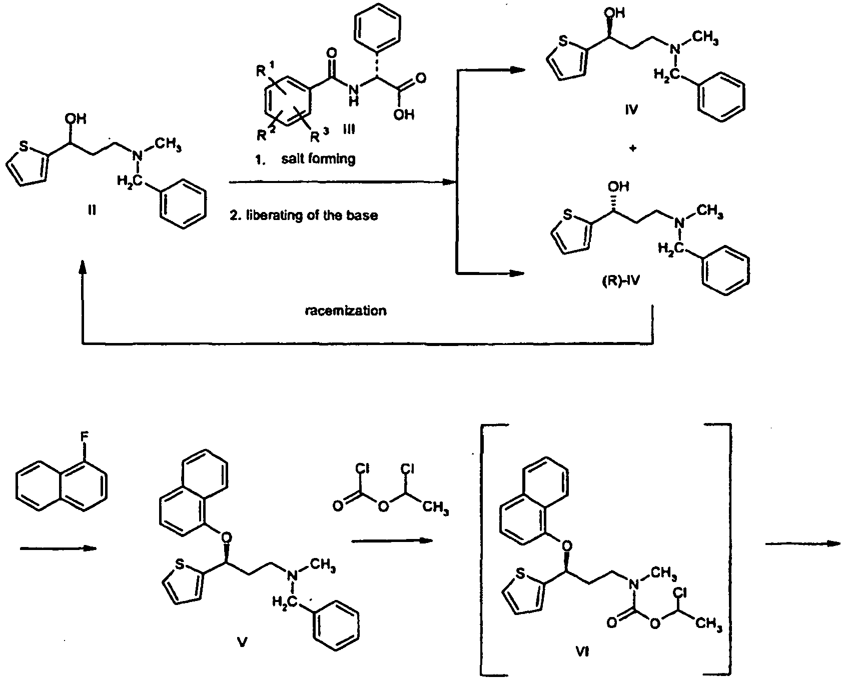

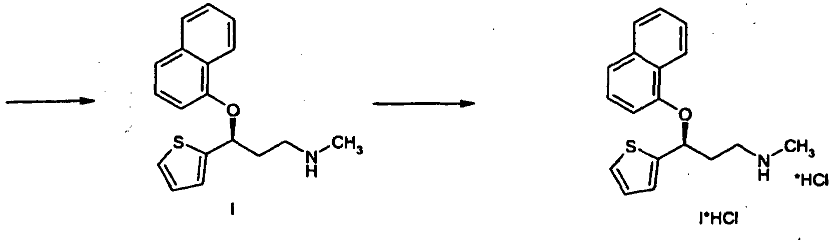

Further objects of the invention are the oxalate salt of (S)-3-(N-methyl-N-benzylamino)-1-(2-thienyl)-1-propanol of formula (IV) and the maleate salt of (S)-N-methyl-N-benzyl-N-(3-(1-naphthalenyloxy)-3-(2-thienyl)propanamine of formula (V) as new key intermediates in the synthesis of duloxetin.

Duloxetin is a combined norepinephrine and serotonin reuptake inhibitor (SNRI), which was developed for the treatment of severe depression as well as for peripheral diabetic neuropathic pain in adults. Owing to its effect on the central nervous system duloxetin decreases parasympathetic activity and increases sympathetic and somatic activity in the lower urinary tract. It results in loosening the bladder and increasing the outflow resistance, therefore duloxetin is suitable for the treatment of SUI (stress urinary incontinence), the most general form of female incontinence.

The synthesis for the preparation of duloxetin and related compounds was originally disclosed in EP 273 658 published European patent application (Eli Lilly and Co.) According to EP Patent No. 273 658 N,N-dimethyl-N-(3-oxo-3-(2-thienyl)propanamine hydrochloride was prepared by Mannich condensation reaction starting from 2-acetyltiophene then the ketene obtained was reduced by sodium borohydride to give 3-dimethylamino-1-(2-thienyl)-1-propanol. The arylation of the secondary hydroxyl group was carried out by reacting it with 1-fluoronaphtalene in the presence of sodium hydride base to give N,N-dimethyl-N-(3-(1-naphthalenyloxy)-3-(2-thienyl)propanamine) which was then demethylated in a two steps procedure. In the first step, N,N-dimethyl-N-(3-(1-naphthalenyloxy)-3-(2-thienyl)propanamine was reacted with phenyl chloroformate to give the corresponding phenylcarbamate. Then the racemic duloxetin was prepared by a basic hydrolysis process performed at high temperature and was isolated in the form of oxalate salt. After resolution of racemic duloxetin by dibenzoyl D-tartaric acid the pure S-enantiomer was obtained. The drawback of the above process is the severe safety risk due to the use of sodium hydride which is extremely fire-hazard. Moreover, carrying out the resolution step at the end of the procedure increases the operation costs.

According to US 5 362 886 published patent application (Eli Lilly and Co.) the resolution step was carried out at an early phase of the synthesis route. Accordingly, 3-dimethylamino-1-(2-thienyl)-1-propanol was resolved by (S)-(+)-mandelic acid then S-(-)-3-dimethylamino-1-(2-thienyl)-1-propanol obtained was arylated by 1-fluoronaphthalene in the presence of sodium hydride and potassium benzoate. However a partial racemization has been reported and the optical purity of (S)-(+)-N,N-dimethyl-N-(3-(1-naphthalenyloxy)-3-(2-thienyl) propanamine obtained was only 91%ee.

The first enantioselective method for the preparation of 3-dialkylamino-1-aryl-1-propanone derivatives was disclosed also by Eli Lilly and Co. in EP 457 559 European published patent application and in Tetrahedron Letters, 31(49), 7101-04 (1990). In this method lithium aluminium hydride/tetrahydrofurane complex (LiAlH₄-(THF)₂ was used as reducing agent, which, applied at an industrial scale involves the same safety risks as the use of sodium hydride in process described in EP 273 658. To ensure enantioselectivity a great excess of (2R,3S)-4-dimethylamino-1,2-diphenyl-3-methyl-2-butanol chiral catalyst was applied. The enantiomer excess of (S)-enantiomer was 80-88%, the 1,3-aminoalcohol obtained was purified by crystallization to give the desired product at about 50% yield. When the process was repeated on a higher scale a significant decrease of the enantioselectivity was observed.

Different processes for the stereoselective reduction of the oxo group have also been published. Numerous patent applications deal with the preparation of (S)-3-methylamino-1-(2-thienyl)-1-propanol wherein (S)-3-methylamino-1-(2-thienyl)-1-propanol is designated as a possible intermediate in the synthesis of duloxetin, since it can be naphthylated in a one-step process to give the desired duloxetin target compound. Accordingly, a number of different chemical processes with different stereoselectivities have been developed for the enantioselective reduction of 3-methylamino-1-(2-thienyl)-1-propanone to obtain (S)-3-methylamino-1-(2-thienyl)-1-propanol. According to WO 2004/020389 and WO 2004/031168 published international patent applications the reduction is carried out by hydrogenation in the presence of Rh and Ru catalysts containing chiral ligands, while WO 2004/013123 published international patent application describes a biochemical method for reduction of 3-methylamino-1-(2-thienyl)-1-propanon. US 2004/0249170 and WO 2004/005307 published patent applications disclose the resolution of racemic 3-methylamino-1-(2-thienyl)-1-propanol. However, reacting (S)-3-methylamino-1-(2-thienyl)-1-propanol with 1-fluoronaphtalene in the presence of a strong base results duloxetin with a modest yield of 10-45%, due to the presence of the secondary amine. Moreover, isolation of the end product has to perform by chromatography according to Chirality 2000, 12, 26-29; J. Lab. Comp Radiopharm, 1955, 36(3), 213-223. Due to the above mentioned disadvantages, (S)-3-methylamino-1-(2-thienyl)-1-propanol can not be taken account as a well applicable intermediate to an economical industrial process.

In a process described in WO 2004/056795 published international patent application, 3-dimethylamino-1-(2-thienyl)-1-propanol is naphthylated by potassium hydroxide base and 18-crown 6 phase transfer catalyst. It is mentioned in the application that the racemization of (S)-N,N-dimethyl-N-(3-(1-naphtalenyloxy)-3-(2-thienyl)propanamine obtained can be forced back, however, the extent of the racemization and the enantiomeric purity of the product are not stated.

According to a further procedure described in WO 2006/027798 published international patent application the undesirable racemization occurring during the naphthylation process can be avoided by carrying out the resolution step after introducing the napthyl group into the molecule. Resolution of N,N-dimethyl-3-(1-naphthalenyloxy)-3-(2-thienyl)propanamine and N-Methyl-N-benzene-N-(3-(1-naphthalenyloxy)-3-(2-(2-thienyl)propanamine by Di-para-toluoyl-L-tartaric acid is claimed, however only the resolution of N,N-dimethyl-3-(1-naphthalenyloxy)-3-(2-thienyl)propanamine is supported by working Example. Owing to the base-sensitiveness of the naphthylated amino alcohol, the (R)-izomer of N,N-dimethyl-3-(1-naphthalenyloxy)-3-(2-thienyl)propanamine remained in the mother liquor is racemized by treatment with a strong base (potassium tert-butoxide) in dimethyl sulfoxide solvent. The racemized N,N-dimethyl-3-(1-naphthalenyloxy)-3-(2-thienyl)propanamine obtained with a yield of 65% is then recycled to the resolution step increasing thereby the yield of the desired isomer. The process according to WO 2006/027798 eliminates the undesirable racemization occuring during the naphthylation procedure, however, resolution by expensive 1-fluoronaphtalene after the naphthylation step makes this process too expensive.

CN 1687057 relates to a method for synthesizing duloxetine, which uses 2-acetylthiophene as initial raw material, and makes it undergo the processes of reaction, reduction and resolution to obtain S)-(-)-3-(N-methyl-N-benzylamino)-1-thiophen-2-yl-1-propanol as an intermediate compound. The intermediate compound is reacted with fluoronaphthalene to obtain the naphtyl derivative, which is then substituted by a halogenated ester and subjected to an elimination reaction under the alkaline condition so as to obtain (S)-(+)-N-methyl-3-(1-naphthoxy)-3-(2-thienyl) propylamine.

EP1506965 relates to the preparation of a racemate or an optically active substance (S- or R-isomer) of a 3-N-methylamino-1-(2-thienyl)-1-propanol compound. The optical resolution is carried out by obtaining a diastereomer salt of the compound with an optically active organic acid, followed by optically resolving the diastereomer salt

a In the light of the disadvantages of the processes listed above, our aim was to provide a process for preparation of duloxetin in industrial scale which is safe and uses economical and convenient reaction steps and by which the active agent can be prepared with the required drug-purity.

Another aim was to provide a process by which the active agent can be prepared without complicated reaction steps or technology requiring extreme conditions and the intermediate compounds can be isolated via simple reaction steps producing good yields and high purity.

Besides, our aim is to carry out the costly reaction steps at the end of the reaction route and to regenerate the expensive (i. e. optical active) ingredients.

In the course of our experiments we have surprisingly found, that in the process according to the invention starting from 3-(N-methyl-N-benzene-amino)-1-(2-thienyl)-1-propanol, a crystalline intermediate namely the maleate salt of (S)-N-methyl-N-benzene-N-(3-(1-naphthalenyloxy)-3-(2-thienyl)propanamine is obtained which can be simply purified by recrystallization to give an excellently pure material both in chemical and optical point of view. Using this intermediate compound the duloxetin can be produced on industrial scale by only a few synthesis steps.

The process according to the invention comprises the following steps:

### Step 1:

3-(N-methyl-N-benzylamino)-1-(2-thienyl)-1-propanol of formula (II) (EP 273 658 and EP 457 559 European published patent applications) is reacted with a D-phenylglycine derivative of general formula (III) wherein R¹, R² and R³ represent independently of each other hydrogen, halogen, cyno, nitro, or amino optionally substituted by one or more C₁₋₄ alkyl groups; trifluoromethyl, C₁-C₄ alkyl, C₁-C₄ alkoxy, -C(=O)-NH₂, C₁-C₄ alkoxycarbonyl, trifluoromethoxy or hydroxy to give (S)-3-(N-methyl-N-benzylamino)-1-(2-thienyl)-1-propanol of formula (IV) which is purified in the form of the oxalate salt thereof

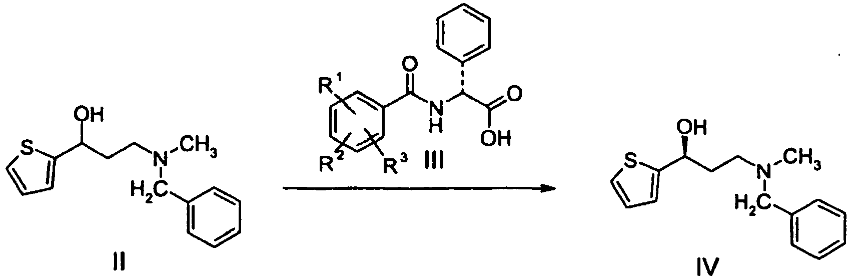

### Step 2:

the (S)-3-(N-methyl-N-benzylamino)-1-(2-thienyl)-1-propanol of formula (IV) obtained in step 1 is reacted with 1-fluoronaphthalene to give (S)-N-methyl-N-benzyl-N-(3-(1-naphthalenyloxy)-3-(2-thienyl)propanamine of formula (V) which is purified in the form of the maleate salt thereof,

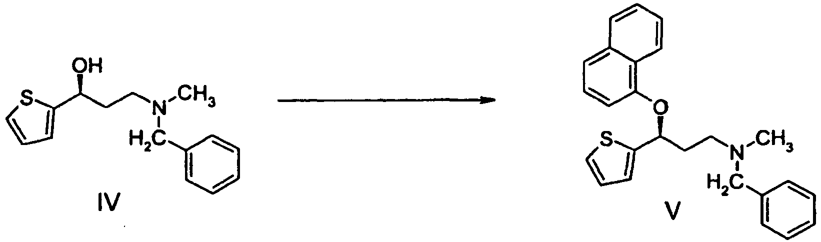

### Step 3:

the (S)-N-methyl-N-benzyl-3-(1-naphthalenyloxy)-3-(2-thienyl)propanamine of formula (V) obtained in step 2 is reacted with 1-chloroethyl chloroformate to give duloxetin ((S)-(+)-N-methyl-3-(1-naphthalenyloxy)-3-(2-thienyl)propanamine) of formula (I) via a non-isolated intermediate compound of formula (VI) 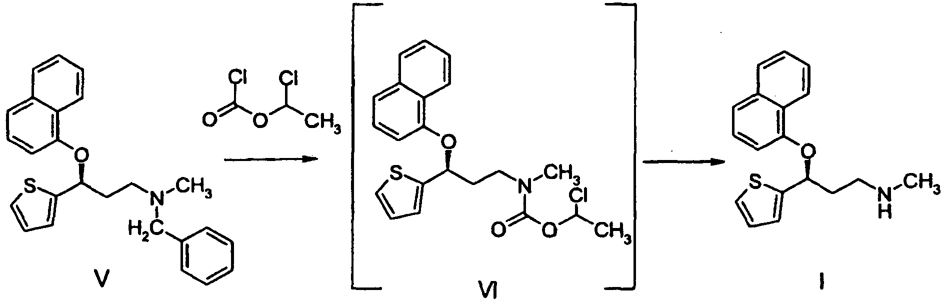 which is purified in the form of the maleate salt thereof,

### step 4:

duloxetin ((S)-(+)-N-methyl-(3-(1-naphthalenyloxy)-3-(2-thienyl)propanamine) of formula (I) obtained in step 3 is converted to its a hydrogen chloride salt in a suitable solvent for example in ethyl acetate or acetone to obtain duloxetin hydrochloride (I*HCl) active agent. Alternatively, duloxetin hydrochloride can be obtained directly by converting (S)-(+)-N-methyl-3-(1-naphthalenyloxy)-3-(2-thienyl)propanamine maleate salt to the desired hydrochloride salt.

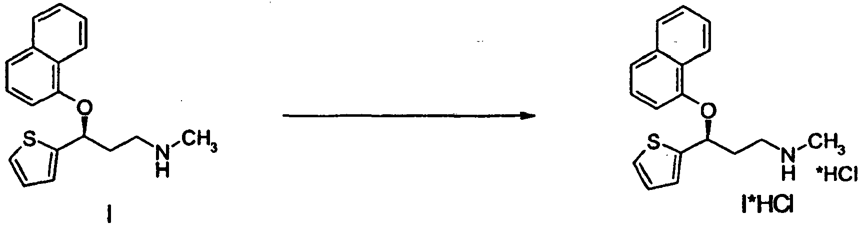

### Detailed description of the invention

### Step 1.

3-(N-methyl-N-benzylamino)-1-(2-thienyl)-1-propanol of formula (II) is resolved by adding 0,5-1 equivalent of a D-phenylglycine derivative of general formula (III) - wherein R¹, R² and R³ represent independently of each other hydrogen, halogen, cyno, nitro, or amino optionally substituted by one or more C₁₋₄ alkyl groups; trifluoromethyl, C₁-C₄ alkyl, C₁-C₄ alkoxy, -C(=O)-NH₂, C₁-C₄ alkoxycarbonyl, trifluoromethoxy or hydroxy - preferably with N-benzoyl D-phenylglycine (R'=H, R²=H, R³=H) in an appropriate organic solvent for example in ethanol, ethyl acetate, tert. butyl methyl ether, acetone, preferably in acetone:

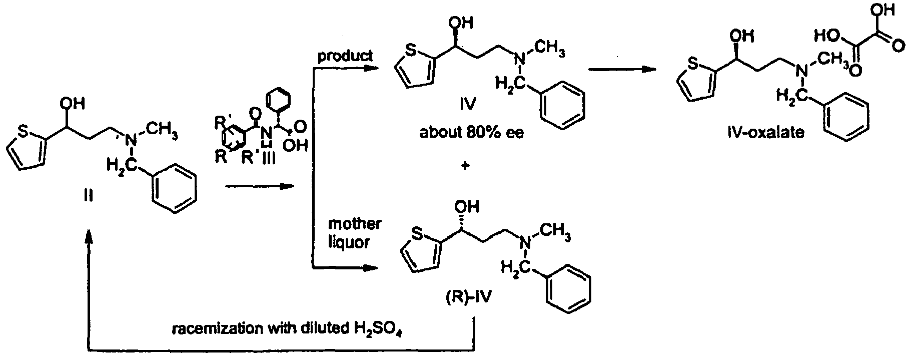

The crystalline diastereomere salt obtained is filtered. Then the diastereomere salt is treated with a mixture of aqueous ammonia or potassium carbonate and a suitable organic solvent, preferably n-hexane. The organic and aqueous layers are separated, the organic layer is evaporated to obtain the free duloxetine base. The resultant 3-(N-methyl-N-benzylamino)-1-(2-thienyl)-1-propanol (the optical purity is about 80% ee for the S-isomer) is reacted with oxalic acid in methanol to give the oxalate salt. After adding tert. butyl methyl ether and filtering the crystalline racemate the methanolic mother liquor is evaporated to give the crystalline (S)-3-(N-methyl-N-benzylamino)-1-(2-thienyl)-1-propanol oxalate salt. Enantiomeric purity of (S)-3-(N-methyl-N-benzylamino)-1-(2-thienyl)-1-propanol obtained is >98%ee. The aquoeus phase is treated with hydrochloric acid and the precipitated D-phenylglycin derivative of general formula (III) (resolving agent) is recycled for further use. The amino alcohol derivative enriched in (R)-3-(N-methyl-N-benzylamino)-1-(2-thienyl)-1-propanol is isolated from the acetone mother liquor and racemised by warming it in aqueous sulphuric acid then the resolution step is repeated.

### Step 2.

In our experiments we have surprisingly found, that the rate of the reaction of 1-fluoronaphtalene with (S)-3-(N-methyl-N-benzylamino)-1-(2-thienyl)-1-propanol in the presence of sodium hydroxide is the same wether a phase transfer catalyst is present or not, thus the presence of a phase transfer catalyst is not necessary. According to our experiments and known literature data the racemization is unavoidable under the naphthylation reaction conditions. The extent of racemization can be decreased by employing aqueous DMSO as a solvent and by decreasing the temperature of the reaction mixture. Accordingly, the naphthylation reaction is carried out as follows. In a mixture of DMSO and tetrahydrofurane containing 20-40%, preferably 40% DMSO, (S)-3-(N-methyl-N-benzylamino)-1-(2-thienyl)-1-propanol of formula (IV) is reacted with 1,1-1,5 mol equivalents, preferably 1,2 mol equivalents of 1-fluoronaphthalene in the presence of 5-10 mol, preferably 7 mol of potassium hydroxide base, at a temperature of 40-45°C. The extent of the predicted racemization is 1-2%, therefore the optical purity of the product is 94-96%ee. The product, (S)-N-methyl-N-benzyl-3-(1-naphthalenyloxy)-3-(2-thienyl)propanamine of formula (V) is isolated in the form of maleate salt after an extraction work-up, then the resulting salt is optionally purified by crystallization. The purification in the form of maleate salt is particularly preferred owing to the fact, that the crystallization of (S)-N-methyl-N-benzyl-(3-(1-naphthalenyloxy)-3-(2-thienyl)propanamine from the oversaturated solution happens in high optical purity while the racemate remains in the mother liquor. The base is liberated from the maleate salt for use in the subsequent step 3. (S)-N-methyl-N-benzyl-3-(1-naphthalenyloxy)-3-(2-thienyl)propanamine of formula (V) obtained has an optical purity of >99,8 %ee.

### Step 3.

In dichloromethane (S)-N-methyl-N-benzyl-3-(1-naphthalenyloxy)-3-(2-thienyl)propanamine of formula (V) is reacted with 1-1,1 mol equivalents of 1-chloroethylformiate in the presence of 0,3-1 equivalent of diisopropyl ethylamine at a temperature of 20-30°C. The carbamate intermediate of formula (VI) obtained is stirred in methanol at a temperature of 20-30°C then the reaction mixture is extracted and the resulting duloxetin ((S)-(+)-N-methyl-3-(1-naphthalenyloxy)-3-(2-thienyl)propanamine) of formula (I) was precipitated from a suitable organic solvent for example from ethyl acetate or tertiary butyl methyl ether in the form of oxalate or maleate salt, preferably in the form of maleate salt thereof. Then the base is liberated from the maleate salt to convert it to duloxetin-hydrochloride salt in the following step.

### Step 4.

(S)-(+)-N-methyl-N-(3-(1-naphthalenyloxy)-3-(2-thienyl)propanamine) of formula (I) is dissolved in an anhydrous aprotic organic solvent preferably in ethyl acetate or acetone, then ethyl acetate containing 0,95-0,98 equivalent of hydrogen chloride or aqueous hydrochloric acid is added at a temperature of 0-25° C to give the hydrochloride salt. The optical purity of duloxetine hydrochloride salt (I*HCl) obtained after filtering and drying is >99.8%ee. Alternatively, the maleate salt of (S)-(+)-N-methyl-N-(3-(1-naphtaleneoxy)-3-(2-thienyl)propanamine of formula (I) obtained in step 3 is suspended in acetone then the resulting duloxetin hydrochloride salt is precipitated by adding cc. hydrochloric acid solution containing 0,95-0,98 equivalent hydrogen chloride.

Advantages of the process according to the invention are as follows:
a) Owing to its excellent crystallization the new (S)-N-methyl-N-benzyl-3-(1-naphthalenyloxy)-3-(2-thienyl)propanamine of formula (V) key intermediate can be purified efficiently in a simple crystallization process. Starting from the new key intermediate compound of formula (V) the duloxetin active agent can be obtained in high chemical and optical purity without using particular reagents and extreme reaction conditions.
b) In the process according to the invention, the resolution is carried out in an early step of the reaction route what enables the production costs to be decreased substantially. In this step a novel intermadiate compund, (S)-3-(N-methyl-N-benzylamino)-1-(2-thienyl)-1-propanol of formula (IV) is obtained by an easy-to-make and economical process.
c) The (R)-3-(N-methyl-N-benzylamino)-1-(2-thienyl)-1-propanol of formula (IV) accumulated in the mother liquor during the resolution procedure is racemized easily then the raceme product obtained is resolved repeatedly.
d) The extent of racemization occurred in the naphthylaton reaction is minimalized by using a suitable solvent mixture.
e) Neither in the resolution step nor in the naphthylation step, the intermediates need not being produced with extra high optical purity since owing to its excellent crystallization properties the key intermediate (S)-N-methyl-N-benzyl-3-(1-naphthalenyloxy)-3-(2-thienyl)propanamine maleate salt can be purified efficiently in a simple crystallization process.

Summing up, the process according to the invention is suitable for economical preparation of duloxetin on industrial scale. Besides the purity of duloxetin active agent produced on the basis of the process according to the invention meets the expected drug-purity requirements.

The present invention provides a process for the preparation of duloxetin

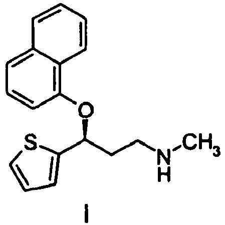

### ((S)-(+)-N-methyl-3-(1-naphthalenyloxy)-3-(2-thienyl)-propanamine) of formula (I) and pharmaceutically acceptable salts thereof by

resolving the raceme 3-(N-methyl-N-benzylamino)-1-(2-thienyl)-1-propanol of formula (II) 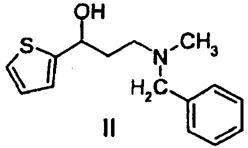 with a D-phenylglycine derivative of general formula (III) 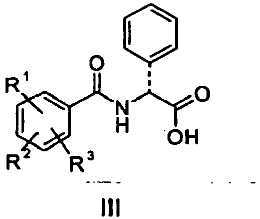 wherein R¹, R² and R³ present independently of each other hydrogen, halogen, cyano, nitro; or amino optionally substituted by one or more C₁-C₄ alkyl groups; trifluoromethyl, C₁-C₄ alkyl, C₁-C₄ alkoxy, -C(=O)-NH₂, C₁-C₄ alkoxycarbonil, trifluoromethoxy or hydroxy to yield (S)-3-(N-methyl-N-benzylamino)-1-(2-thienyl)-1-propanol of formula (IV) 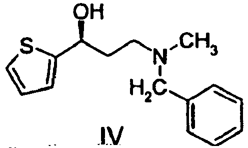 purifying the compound of formula (IV) in the form of the oxalate salt thereof and reacting the compound of formula (IV) obtained with 1-fluoronaphthalene to yield (S)-N-methyl-N-benzyl-3-(1-naphthalenyloxy)-3-(2-thienyl)propanamine of formula (V) 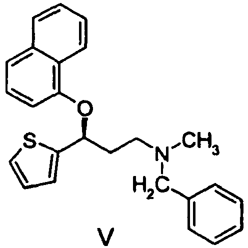 reacting the compound of formula (V) obtained with (1-chloroethyl) chloroformate and optionally converting the duloxetin of formula (I) obtained to a pharmaceutically salt thereof.

In a preferred embodiment of the invention the resolution step is carried out with N-benzoyl D-phenylglycine.

In another preferred embodiment of the invention the purification of (S)-3-(N-methyl-N-benzylamino)-1-(2-thienyl)-1-propanol of formula (IV) is performed by fractioning crystallization, preferably in the form of its oxalate salt. In a further preferred embodiment of the invention the purification of (S)-N-methyl-N-benzyl-3-(1-naphthalenyloxy)-3-(2-thienyl)propanamine of formula (V) is carried out also by fractioning crystallization, preferably in the form of its maleate salt.

In a further preferred embodiment of the invention the (S)-(+)-N-methyl-3-(1-naphthalenyloxy)-3-(2-thienyl)propanamine of formula (I) is isolated in the form of its maleate salt then either the is base liberated in solution and then reacted with anhydrous hydrogen chloride without isolation to give duloxetin.HCl active agent, or without liberating the base, the maleate salt is reacted with cc. aqueous hydrochloric acid to give duloxetin.HCl active agent.

Moreover, the present invention relates to the oxalate salt of (S)-N-methyl-N-benzylamino)-1-(2-thienyl)-1-propanol of formula (IV). Another object of the invention is a process for the preparation of (S)-3-(N-methyl-N-benzylamino)-1-(2-thienyl)-1-propanol of formula (IV) 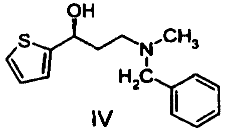 comprising resolving raceme 3-(N-methyl-N-benzylamino)-1-(2-thienyl)-1-propanol of formula (II) 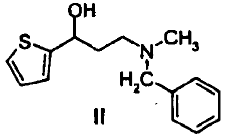 with a D-phenylglycin derivative of general formula (III) 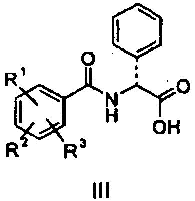 wherein R¹, R² and R³ present independently of each other hydrogen, halogen, ciano, nitro, or amino optionally substituted with one or more C₁-C₄ alkyl group; trifluoromethyl, C₁-C₄ alkyl, C₁-C₄ alkoxy, -C(=O)-NH₂, C₁-C₄ alkoxycarbonil, trifluoromethoy or hydroxy.

In a preferred embodiment of the invention the resolution step is carried out by N-benzoyl D-phenylglycine.

In another preferred embodiment of the invention the purification of (S)-3-(N-methyl-N-benzylamino)-1-(2-thienyl)-1-propanol of formula (IV) is performed by fractioning crystallization, preferably in the form of its oxalate salt.

The present invention further relates to the maleate salt of (S)-N-methyl-N-benzyl-3-(1-naphthalenyloxy)-3-(2-thienyl)propanamine of formula (V).

Besides, the present invention relates to a process for the preparation of (S)-N-methyl-N-benzyl-3-(1-naphthalenyloxy)-3-(2-thienyl)propanamine of formula (V) comprising 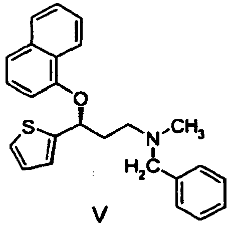 resolving raceme 3-(N-methyl-N-benzylamino)-1-(2-thienyl)-1-propanol of formula (II) 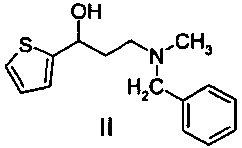 with a D-phenylglycine derivative of general formula (III) 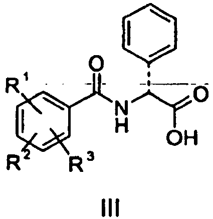 wherein R¹, R² and R³ present independently of each other hydrogen, halogen, cyano, nitro or amino optionally substituted with one or more C₁-C₄ alkyl groups; trifluoromethyl; C₁-C₄ alkyl, C₁-C₄ alkoxy, -C(=O)-NH₂, C₁-C₄ alkoxycarbonil, trifluoromethoxy or hydroxy, and reacting (S)-3-(N-methyl-N-benzylamino)-1-(2-thienyl)-1-propanol of formula (IV) 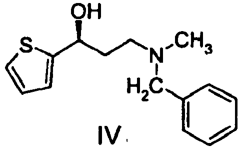 obtained above with 1-fluoronaphthalene.

In a preferred embodiment of the above process, the resolution is carried out with N-benzoyl D-phenylglycine.

In another preferred embodiment of the process, the purification of (S)-3-(N-methyl-N-benzylamino)-1-(2-thienyl)-1-propanol of formula (IV) is carried out by fractioning crystallization, preferably in the form of the oxalate salt thereof.

In a further preferred embodiment of the process the purification of (S)-N-methyl-N-benzyl-3-(1-naphthalenyloxy)-3-(2-thienyl)propanamine of formula (V) is carried out by fractioning crystallization, preferably in the form of the maleate salt thereof.

The analytical methods used in the specification are as follows.

To identify the compounds of the invention ¹H NMR spectra were made by Varian UNITY *INOVA* 500 spectrometer.

The chemical displacement values are stated in ppms (parts per million), comparing to tetramethylsilane internal standard.

The optical purity of the optically active intermediate compounds was determined by chiral HPLC methods.
*1.* The purity of (S)-3-(N-metil-N-benzil-amino)-1-(2-tienil)-1-propanol of formula (IV) was determined by HPLC as follows:
   Column: CHIRALPAK IA (Chiral Technologies Europe) 250x4.6 mm 5µm
   Temperature: 25°C
   Eluent: n-hexane : chloroform : diethyl amine = 92 : 8 : 0,1
   Flow rate: 1 ml/min
   Detection: UV detector, λ (wave length) = 254 nm
   Approximate retention times: for (S)-3-(N-methyl-N-benzylamino)-1-(2-thienyl)-1-propanol: 19,9 min; for
   (R)-3-(N-methyl-N-benzylamino)-1-(2-thienyl)-1-propanol: 23,1 min.
2. The purity of (S)-N-methyl-N-benzyl-N-(3-(1-naphthalenyloxy)-3-(2-thienyl)propanamine of formula (V) was determined by HPLC as follows:
   Column: CHIRALPAK IB (Chiral Technologies Europe) 250x4.6 mm 5µm
   Temperature: 25°C
   Eluent: n-hexane:2-propanol=99,5:0,5 (+ 0,1% diethyl amine)
   Flow rate: 1 ml/min
   Detection: UV detector, λ =255 nm,
   Approximate retention times: for (S)-N-methyl-N-benzyl-N-(3-(1-naphthalenyloxy)-3-(2-thienyl)propanamine: 7,7 min, for (R)-N-methyl-N-benzyl-N-(3-(1-naphthalenyloxy)-3-(2-thienyl)propanamine: 11,4 min.
   (S)-N-methyl-N-benzyl-N-(3-(1-naphthalenyloxy)-3-(2-thienyl)propanamine maleate salt melting point: 145-147°C
3. The purity of (S)-N-methyl-N-(3-(1-naphthalenyloxy)-3-(2-thienyl)propanamine of formula (I) was determined by HPLC as follows:
   Column: CHIRALPAK IB (Chiral Technologies Europe) 250x4.6 mm 5µm
   Temperature: 35°C
   Eluent: n-hexane:2-propanol:ethanol=95:4,5:0,5 (+ 0,3% diethyl amine)
   Flow rate: 1 ml/min
   Detection: UV detector, λ =254 nm
   Approximate retention times: for (S)-N-methyl-N-(3-(1-naphthalenyloxy)-3-(2-thienyl)propanamine: 11,2 min; for (R)-N-methyl-N-(3-(1-naphthalenyloxy)-3-(2-thienyl)propanamine: 14,0 min.

The invention is illustrated by the following non-limiting Examples:

### Example 1

### Preparation of (S)-3-(N-methyl-N-benzylamino)-1-(2-thienyl)-1-propanol of formula (IV)

### Resolution method A

60 g (0,23 mol) of 3-(N-methyl-N-benzylamino)-1-(2-thienyl)-1-propanol was dissolved in 300 cm³ of acetone and the solution was heated to boiling point To the base solution obtained a mixture of 29,3 g (0,115 mol) of N-benzoyl D-phenylglicine and 6,5 cm³ (0,115 mol) of acetic acid dissolved in 600 cm³ of hot acetone was added. The resulting suspension was boiled for 30 minutes then, after cooling to 20-25°C temperature, the mixture was further stirred for 1 hour, filtrated and washed with acetone.
Dry weight: 50 g (84%)

The salt obtained in the foregoing step was suspended in the mixture of 700 cm³ of n-hexane and 700 cm³ of water. To the resulting suspension 20 cm³ of ammonia solution (25%) was added then the mixture was stirred to get two homogenous layers. The layers were separated and the aqueous layer was extracted with n-hexane (2x300 cm³). Combined organic extracts were washed with water (1x300 cm³) then dried over sodium sulphate and evaporated.
Dry weight of the oil obtained is 25 g (83%), the optical purity is 65-70%ee.

Repeating the resolution process by using 0,8 equivalent of resolving agent 18,1 g of the title compound was obtained with optical purity of >80%ee.

The pH of combined aqueous layers was adjusted to pH 2 by adding hydrochloric acid solution (2M) and the crystalline N-benzoyl D-phenylglycine obtained was filtered, neutralized by washing with deionizated water and dried to give 22 g of N-benzoyl D-phenylglyicine (90% of the N-benzoyl D-phenylglyicine present in the diastereomere salt).

### Resolution method B

To a solution of 2,6 g (0,01 mol) of 3-(N-metil-N-benzil-amino)-1-(2-tienil)-1-propanol in 13 cm³ of acetone 1,3 cm³ of water, 0,1 cm³ of cc. sulphuric acid solution and 1,53 g (0,006 mol) of N-benzoyl-D-phenylglycine were added. The reaction mixture was heated to boiling point and after the dissolution was complete, it was cooled to 20-25 °C temperature. The crystalline suspension was stirred for 20 hours then filtered and washed with acetone (2x5 cm³) to afford 2,2 g (85%) of the title compound (75-80%ee).

### Purifying in the form of oxalate salt

To a solution of 17,8 g (0,068 mol) of (S)-3-(N-methyl-N-benzyl-amino)-1-(2-thienyl)-1-propanol (80 %ee) in 180 cm³ of methanol, anhydrous oxalic acid (6,1 g, 0,068 mol) was added and the reaction mixture was stirred at 20-25°C temperature for 1 hour. The obtained crystalline oxalate salt (about 6 g) was filtered, the filtrate was evaporated to about 30 cm³, then 90 cm³ of tert. butyl methyl ether was added to yield purified (S)-3-(N-methyl-N-benzylamino)-1-(2-thienyl)-1-propanol oxalate salt as crystalls. The crystalline material obtained was filtered, washed with tert. butyl methyl ether and dried.
Yield: 16,6 g (69%).
The base was liberated from the oxalate salt in the manner descibed in method **A**. The optical purity of the obtained (S)-3-(N-methyl-N-benzylamino)-1-(2-thienyl)-1-propanol is >98 %ee.
¹H NMR (500 MHz, DMSO-*d₆*, 25 °C) δ 2,00-2,15 (m, 2H), 2,57 (s, 3H), 2,92-3,11 (m, 2H), 4,06-4,24 (m, 2H), 4,88 (dd, *J* = 7,6, 4,9 Hz, 1H), 6,93-6,99 (m, 2H), 7,37-7,50 (m, 6H) ppm.

### Racemization by starting from the mother liquor enriched in R-3-(N-methyl-N-benzylamino)-1-(2-thienyl)-1-propanol

About 1 dm³ of the acetone mother liquor obtained in the resolution step was evaporated in vacuum and the residue was dissolved in 300 cm³ of 1M sulphuric acid. The insoluble N-benzoyl D-phenyl-glycine resolving agent was precipitated in crystalline form then it was filtered off and in a 500 cm³ flask, the filtrate was refluxed at 50°C temperature under stirring. The progress of the reaction was followed by chiral HPLC. Completing the racemization process requires about 1,5-2 hours. After completion of racemization the reaction mixture was cooled to a temperature between 20-25°C and 100 cm³ of n-hexane was added followed by dropwise addition of 500 cm³ of 10% potassium carbonate solution under stirring. The layers were separated and the aqueous layer was extracted with n-hexane (2× 100 cm³). The combined organic layers were dried over sodium sulphate, the sodium sulphate was filtered off and the filtrate was evaporated under vacuum.

The resulting 34 g of raceme 3-(N-methyl-N-benzyl amino)-1-(2-thienyl)-1-propanol can be purified in the form of the oxalate salt thereof. Accordingly, 34 g of raceme 3-(N-methyl-N-benzylamino)-1-(2-thienyl)-1-propanol was dissolved in 150 cm³ of tert. butyl methyl ether and 15 cm³ of methanol was added. Then the mixture was cooled to 0-5°C temperature. To the cooled mixture a solution of 11,7 g of anhydrous oxalic acid and 30 cm³ of methanol was added slowly, under stirring meanwhile the mixture was cooled by ice-water. The precipitated oxalate salt was filtered through a glass filter, washed with 2 x 20 cm³ of tert. butyl methyl ether, then dried in an open vessel under room temperature to obtain a white crystalline powder.
Weight: 45 g

### Example 2.

### Preparation of (S)-N-methyl-N-benzyl-3-(1-naphthalenyloxy)-3-(2-thienyl)propanamine

A 250 cm³ flask equipped with a magnetic stirrer and a thermometer was charged with 10,4 g (0,04 mol) of (S)-3-(N-methyl-N-benzylamino)-1-(2-thienyl)-1-propanol, 15 cm³ of DMSO, 45 ml of tetrahydrofurane, 15,7 g (0,28 mol, 7 equivalents) of powdered potassium hydroxide and 5,3 cm³ (0,048 mol), 1,2 equivalents) of 1-fluoronaphthalene, and the mixture was stirred at a temperature between 40-45°C for 9-12 hours. The progress of the reaction was followed by thin-layer chromatography. After completion of the reaction the mixture was cooled to a temperature between 20-25°C and poured into 150 cm³ of ice-water then extracted with n-hexane (3x50 cm³). The combined organic phases were washed with water (3x50 ml), dried over Na₂SO₄ and evaporated under vacuum to give the crude title compound.
Weight: 17,0 g, optical purity >95 %ee by chiral HPLC.

### Preparation of (S)-N-methyl-N-benzyl-3-(1-naphthalenyloxy)-3-(2-thienyl)propanamine maleate

17,0 g of crude (S)-N-methyl-N-benzyl-3-(1-naphthalenyloxy)-3-(2-thienyl)propanamine base was dissolved in 150 ml of ethyl acetate, and 4,7 g (0,04 mol) of maleic acid was added. The precipitated crystals were stirred for 1 hour then filtered and washed with ethyl acetate.
Weight of the crystalline maleate salt: 16,2 g (0,032 mol) (85%), optical purity >99 %ee by chiral HPLC method.

### Recrystallization of the (S)-N-methyl-N-benzyl-3-(1-naphthalenyloxy)-3-(2-thienyl)propanamine salt

16,2 g (0,032 mol) of (S)-N-methyl-N-benzyl-N-(3-(1-naphthalenyloxy)-3-(2-thienyl)propanamine maleate salt obtained in the previous step was dissolved in 90 ml of hot ethanol and the reaction mixture was cooled to 0°C temperature for about 30 minutes. It was stirred at 0°C temperature for further 30 minutes, the precipitated crystals was filtered and washed with cold ethanol.
Dried weight: 14,5 g (90%)
¹H NMR (500 MHz, DMSO-*d₆*, 25 °C) δ 2,36-2,54 (m, 1H), 2,55-2,69 (m, 1H), 2,73 (s, 3H), 3,10-3,43 (brm, 2H), 4,33 (brs, 2H), 6,02 (dd, *J* = 7,8, 4,9 Hz, 1H), 6,07 (s, 2H), 6,98-7,03 (m, 2H), 7,23 (dm, *J =* 3,5 Hz, 1H), 7,34 (t, *J =* 7,8 Hz, 1H), 7,39-7,58 (m, 9H), 7,83-7,89 (m, 1H), 8,12-8,20 (m, 1H), 9,77 (vbrs, 1H), 19,03 (vbrs, 1H) ppm.

### Liberating (S)-N-methyl-N-benzyl-3-(1-naphthalenyloxy)-3-(2-thienyl)propanamine base from the maleate salt thereof

14,5 g (0,029 mol) of (S)-N-methyl-N-benzyl-N-(3-(1-naphthalenyloxy)-3-(2-thienyl)propanamine maleate was suspended in a mixture of 200 cm³ of dichloromethane and 250 cm³ of a potassium chloride solution (10%), and the mixture was stirred until complete dissolution of the maleate salt. The layers were separated, the aqueous layer was extracted with dichloromethane (2x20 cm³) and the combined organic layers were washed with 2x30 cm³ of water. The dichloromethane solution was dried over sodium sulphate and evaporated to obtain the title compound in an oily form.
Yield: 11,0 g (0,028 mol), optical purity >99,5%ee by chiral HPLC method.

### Example 3.

### Preparation of (S)-N-methyl-3-(1-naphthalenyloxy)-3-(2-thienyl)propanamine maleate (duloxetin-maleate)

Under room temperature a 250 cm³ flask equipped with a magnetic stirrer and a thermometer was charged with 13,6 g (0,035 mol) of (S)-N-methyl-N-benzyl-(3-(1-naphthalenyloxy)-3-(2-thienyl)propanamine, 150 cm³ of dichloromethane and 7,4 cm³ (0,042 mol) of diisopropyl-ethyl-amine then 4,5 cm³ (0,042 mol) of 1-chloroethyl chloroformate was added dropwise. The reaction mixture was stirred at 25°C temperature for about 2 hours meanwhile the progress of the reaction was followed by thin-layer chromatography. After completion of the reaction the mixture was poured into 150 cm³ of citric acid solution (5%) and the layers were separated. The aqueous layer was extracted with 100 cm³ of dichloromethane then the combined organic layers were washed with 100 cm³ of water until they were acid-free. The organic layer was dried over Na₂SO₄, dried under vacuum to yield 16,0 g of crude carbamate (VI).

In a 250 cm³ flask equipped with a magnetic stirrer and a gas bubbling apparatus the crude carbamate (VI) was dissolved in 80 cm³ of methanol and was stirred until gas formation occurred. The progress of the reaction was followed by thin-layer chromatography. After completion of the reaction 80 cm³ of water was added to the mixture and the solution obtained was extracted with 2 x 50 cm³ of n-hexane. The methanol-water layer was alkalized with 30 cm³ of a potassium carbonate solution (10%) then the mixture was extracted with 2 x 200 cm³ of ethyl acetate. The combined organic layers were washed with about 100 ml of water, dried over Na₂SO₄ and evaporated under vacuum to about 80 cm³ volume. Then 3,3 g (0,0366 mol) of maleic acid was added to the mixture under stirring. The maleate salt obtained was stirred for about 1 hour, filtered, and washed with 2x20 cm³ of ethyl acetate then dried under vacuum to give 10,9 g of crystalline duloxetin-maleate.
Yield: 74%.

The free duloxetin base can be obtained from the maleate salt thereof by using the procedure described in Example 2.

### Example 4.

### Preparation of (S)-N-methyl-3-(1-naphthalenyloxy)-3-(2-thienyl)propanamine chloride (duloxetin-hydrochloride)

### Salt forming with cc. aqueous hydrochloric acid solution in an organic solvent

A 250 cm³ flask equipped with a magnetic stirrer was charged with 4,0 g (0,0097 mol) of (S)-N-methyl-N-(3-(1-naphthalenyloxy)-3-(2-thienyl)propanamine maleate, 100 cm³ of potassium carbonate solution (10%) and 100 cm³ of n-hexane. The heterogeneous mixture was stirred under room temperature until complete dissolution of the maleate salt, then the layers were separated and the aqueous layer was extracted with 2x50 cm³ of n-hexane. The combined organic layers were washed with 1 x50 cm³ of water to become neutral. The organic layer was dried over 20 g of sodium sulphate then dried in vacuum. The residual 2,9 g (0,0097 mol) of duloxetin base was dissolved in 29 cm³ of acetone and the base solution obtained was cooled to 0-5° C temperature by ice-water and 0,8 cm³ (0,0097 mol) of hydrochloric acid (37%) was added dropwise. Then the mixture was seeded with seed crystals, and it was stirred at a temperature between 0-5° C for about 1-2 hours. The precipitated crystals were filtered and washed with 2x5 cm³ of acetone then was air-dried to obtain 2,65 g of crystalline duloxetin hydrochloride (82%).

### Salt forming with anhydrous hydrogen in an organic solvent

1,0 g (0,0033 mol) of duloxetin base was dissolved in 8 cm³ of ethyl acetate and under room temperature 1,0 cm³ of ethyl acetate containing 0,0033 mol of dry hydrogen chloride was added dropwise. After starting crystallization the suspension was stirred for 15 minutes, filtered and washed with 2 x 1 cm³ of ethyl acetate to obtain 0,71 g of crystalline duloxetin hydrochloride, yield: 64,5%.

### Preparation of the hydrochloride salt starting from the maleate salt.

0,82 g (0,002 mol) of duloxetin maleate was suspended in 8 cm³ of acetone and cooled to a temperature between 0-5° C. To the cooled suspension 0,17 cm³ (0,002 mol) of cc. hydrochloric acid solution was added dropwise. After dissolution of the maleate salt the duloxetin hydrochloride salt was precipitated. The crystalline suspension was stirred at a temperature between 0-5°C for 1-2 hours then filtered, washed with 2 x 2 cm³ of acetone and was air dried to obtain 0,42 g of (64%) crystalline duloxetin hydrochloride.
¹H NMR (500 MHz, DMSO-*d₆*, 25 °C) δ 2,33-2,44 (m, 1H), 2,56 (s, 3H), 2,53-2,64 (m, 1H), 3,00-3,15 (m, 2H), 6,16 (dd, *J =* 7,6, 5,0 Hz, 1 H), 6,99 (dd, *J =* 5,0, 3,5 Hz, 1H), 7,08 (dm, *J =* 7,8 Hz, 1H), 7,28 (dm, *J*= 3,5 Hz, 1H), 7,34 (t, *J*= 7,8 Hz, 1H), 7,43-7,48 (m, 2H), 7,50-7,56 (m, 2H), 7,82-7,89 (m, 1H), 8,23-8,30 (m, 1H), 9,25 (brs, 2H) ppm.

## Claims

1. Process for the preparation of duloxetin 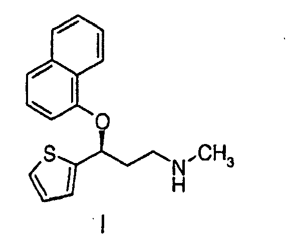 ((S)-N-methyl-3-(1-naphthalenyloxy)-3-(2-thienyl)propanamine) of formula (I) and pharmaceutically acceptable salts thereof, said process comprising
resolving racemic 3-(N-methyl-N-benzylamino)-1-(2-thienyl)-1-propanol of formula (II) 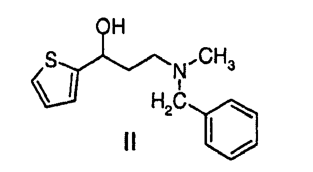 with a D-phenylglycine derivative of general formula (III) 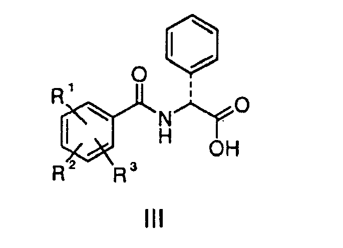 wherein R¹, R² and R³ represent independently of each other hydrogen, halogen, cyano, nitro or amino optionally substituted by one or more C₁-C₄ alkyl groups; trifluoromethyl, C₁-C₄ alkyl, C₁-C₄ alkoxy, -C(=O)-NH₂, C₁-C₄ alkoxycarbonyl, trifluoromethoxy or hydroxy to yield (S)-3-(N-methyl-N-benzylamino)-1-(2-thienyl)-1-propanol of formula (IV) 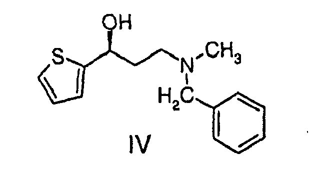 purifying the compound of formula (IV) in the form of the oxalate salt thereof reacting the compound of formula (IV) obtained with
1-fluoronaphthalene to yield (S)-N-methyl-N-benzyl-3-(1-naphthalenyloxy)-3-(2-thienyl)propanamine of formula (V) 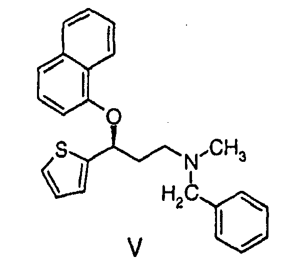 purifying the compound of formula (V) in the form of the maleate salt thereof
reacting the compound of formula (V) obtained with 1-chloroethyl chloroformate to yield duloxetin of formula (I), and
if desired converting the duloxetin of formula (I) obtained into a pharmaceutically acceptable salt thereof.

2. A process according to claim 1 **characterized in that** the resolution is carried out with N-benzoyl D-phenylglycine.

3. The oxalate salt of (S)-3-(N-methyl-N-benzylamino)-1-(2-thienyl)-1-propanol.

4. Process for the preparation of (S)-3-(N-methyl-N-benzylamino)-1-(2-thienyl)-1-propanol of formula (IV) 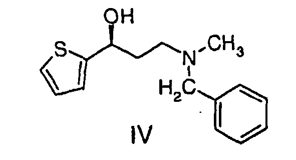 and acid addition salts thereof **characterized in that** racemic 3-(N-methyl-N-benzylamino)-1-(2-thieny)-1-propanol of formula (II) 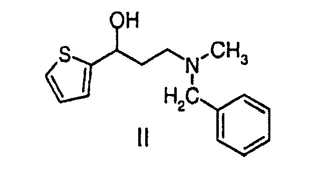 is resolved with a D-phenylglycin derivative of general formula (III) 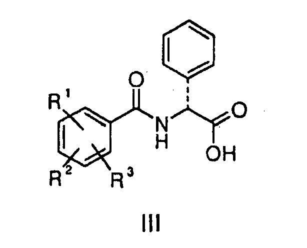 wherein R¹, R² and R³ represent independently of each other hydrogen, halogen, cyano, nitro or amino optionally substituted by one or more C₁-C₄ alkyl groups; trifluoromethyl, C₁-C₄ alkyl, C₁-C₄ alkoxy, -C(=O)-NH₂, C₁-C₄ alkoxycarbonyl, trifluoromethoxy or hydroxy to yield (S)-3-(N-methyl-N-benzylamino)-1-(2-thienyl)-1-propanol of formula (IV), which is purified in the form of the oxalate salt thereof, and
if desired the compound of formula (IV) obtained is precipitated in the form of an acid addition salt thereof.

5. The maleate salt of (S)-N-methyl-N-benzyl-3-(1-naphthalenyloxy)-3-(2-thienyl)propanamine.

6. A process for the preparation of (S)-N-methyl-N-benzyl-3-(1-naphthalenyloxy)-3-(2-thienyl)propanamine of formula (V) 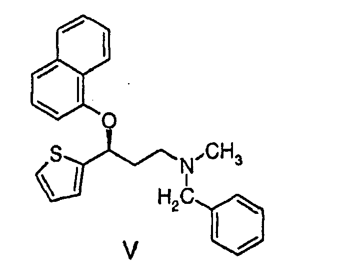 and acid addition salts thereof **characterized in that** 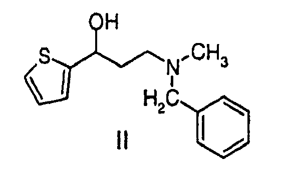 racemic 3-(N-methyl-N-benzylamino)-1-(2-thieny)-1-propanol of formula (II) is resolved with a D-phenylglycine derivative of general formula (III) 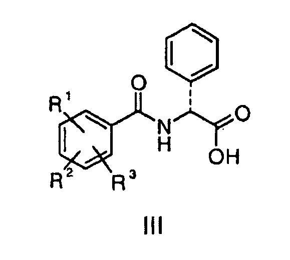 wherein R¹, R² and R³ represent independently of each other hydrogen, halogen, cyano, nitro or amino optionally substituted by one or more C₁-C₄ alkyl groups; trifluoromethyl, C₁-C₄ alkyl, C₁-C₄ alkoxy, -C(=O)-NH₂, C₁-C₄ alkoxycarbonyl, trifluoromethoxy or hydroxy and the (S)-3-(N-methyl-N-benzyl-amino)-1-(2-thienyl)-1-propanol of formula (IV) obtained 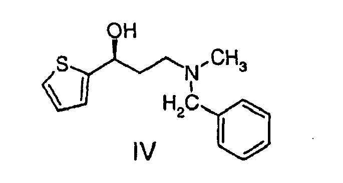 is purified in the form of the oxalate salt thereof and
is reacted with 1-fluoronaphthalene to yield (S)-N-methyl-N-benzyl-3-(1-naphthalenyloxy)-3-(2-thienyl)propanamine of formula (V), which is purified in the form of the oxalate salt thereof, and
if desired the compound of formula (V) obtained is precipitated in the form of an acid addition salt thereof.

7. A process according to claim 4 or claim 6, **characterized in that** the resolution is carried out with N-benzoyl D-phenylglycine.

8. A process according to any one of claim 1 and claim 2 **characterized in that** the duloxetin ((S)-(+)-N-methyl-N-(3-(1-naphthalenyloxy)-3-(2-thienyl)propanamine)) of formula (I) is purified by fractioning crystallization.

9. A process according to claim 8 **characterized in that** the duloxetin ((S)-(+)-N-methyl-3-(1-naphthalenyloxy)-3-(2-thienyl)propanamine of formula (I) is precipitated in the form of the maleate salt thereof then the maleate salt obtained is converted to duloxetin ((S)-(+)-N-methyl-3-(1-naphthalenyloxy)-3-(2-thienyl)propanamine of formula (I).

10. A process according to any one of claim 1 and claim 2 **characterized in that** the duloxetin ((S)-(+)-N-methyl-N-(3-(1-naphthalenyloxy)-3-(2-thienyl)propanamine) of formula (I) is precipitated in the form of the maleate salt thereof then after dissolution of the maleate salt obtained the base is liberated and reacted without isolation with anhydrous hydrogen chloride to yield the duloxetin hydrochloride active agent.

11. A process according to any one of claim 1 and claim 2 **characterized in that** the (S)-(+)-N-methyl-3-(1-naphthalenyloxy)-3-(2-thienyl)propanamine of formula (I) is isolated in the form of the maleate salt thereof then, without liberating the base, the maleate salt obtained is reacted with concentrated aqueous hydrochloric acid in an organic solvent to yield the duloxetin hydrochloride active agent.

12. A process according to any of claims 1, 2, 4, 6 and 7 **characterized in that** (S)-3-(N-methyl-N-benzylamino)-1-(2-thienyl)-1-propanol of formula (IV) is purified by fractioning crystallization.

13. The process according to claim 12 **characterized in that** the (S)-3-(N-methyl-N-benzylamino)-1-(2-thienyl)-1-propanol of formula (IV) is precipitated in the form of the oxalate salt thereof then the oxalate salt obtained is converted to (S)-3-(N-methyl-N-benzylamino)-1-(2-thienyl)-1-propanol of formula (IV).

14. A process according to any one of claims 1, 2 and 6 **characterized in that** the (S)-N-methyl-N-benzyl-3-(1-naphthalenyloxy)-3-(2-thienyl)propanamine of formula (V) is purified by fractioning crystallization.

15. A process according to claim 14 **characterized in that** the (S)-N-methyl-N-benzyl-3-(1-naphthalenyloxy)-3-(2-thienyl)propanamine of formula (V) is precipitated in the form of the maleate salt thereof then the maleate salt obtained is converted to (S)-N-methyl-N-benzyl-3-(1-naphthalenyloxy)-3-(2-thienyl)propanamine of formula (V).

## Patentansprüche

1. Verfahren zur Herstellung von Duloxetin 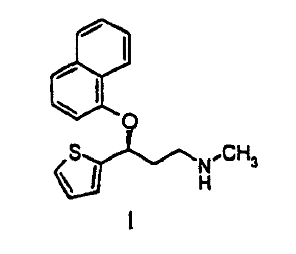 ((S)-N-Methyl-3-(1-naphthyloxy)-3-(2-thienyl)propanamin) der Formel (I) und pharmazeutisch annehmbarer Salze davon, worin das Verfahren umfasst:
Aufspalten von razemischem 3-(N-Methyl-N-benzylamino)-1-(2-thienyl)-1-propanol der Formel (II) 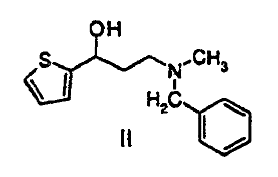
mit einem D-Phenylglycinderivat der allgemeinen Formel (III) 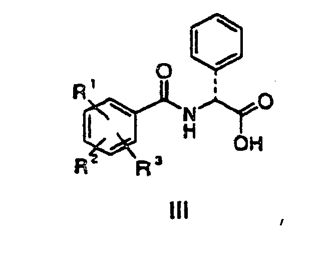
worin R¹, R² und R³ unabhängig voneinander Wasserstoff, Halogen, Cyano, Nitro oder Amino, optional substituiert mit ein oder mehreren C₁₋₄-Alkylgruppen; Trifluoromethyl, C₁₋₄-Alkyl, C₁₋₄-Alkoxy, -C(=O)-NH₂, C₁₋₄-Alkoxycarbonyl, Trifluoromethoxy oder Hydroxy darstellen, um (S)-3-(N-Methyl-N-Benzylamino)-1-(2-thienyl)-1-propanol der Formel (IV) zu ergeben 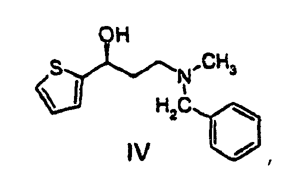
Reinigen der Verbindung der Formel (IV) in Form ihres Oxalatsalzes,
Umsetzen der erhaltenen Verbindung der Formel (IV) mit 1-Fluoronaphthalin, um (S)-N-Methyl-N-Benzyl-3-(1-naphthyloxy)-3-(2-thienyl)propanamin der Formel (V) zu ergeben 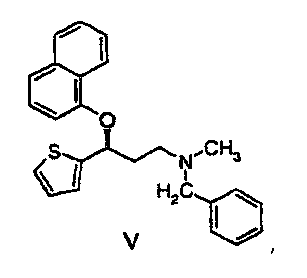
Reinigen der Verbindung der Formel (V) in Form ihres Maleatsalzes,
Umsetzen der erhaltenen Verbindung der Formel (V) mit 1-Chloroethylchloroformat, um Duloxetin der Formel (I) zu ergeben, und
falls gewünscht, Umwandeln des erhaltenen Duloxetin der Formel (I) in ein pharmazeutisch annehmbares Salz davon.

2. Verfahren gemäß Anspruch 1, **dadurch gekennzeichnet, dass** die Aufspaltung mit N-Benzoyl-D-phenylglycin durchgeführt wird.

3. Das Oxalatsalz von (S)-3-(N-Methyl-N-benzylamino)-1-(2-thienyl)-1-propanol.

4. Verfahren zur Herstellung von (S)-3-(N-Methyl-N-benzylamino)-1-(2-thienyl)-1-propanol der Formel (IV) 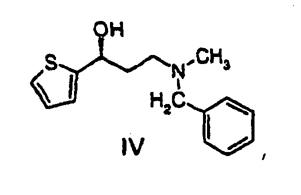 und Säureadditionssalzen davon, **dadurch gekennzeichnet, dass** razemisches 3-(N-Methyl-N-benzylamino)-1-(2-thienyl)-1-propanol der Formel (II) 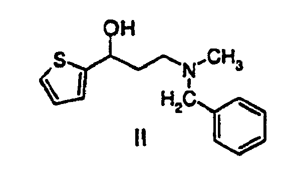 mit einem D-Phenylglycinderivat der Formel (III) aufgespalten wird 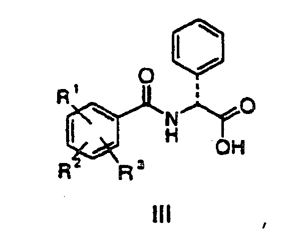 worin R¹, R² und R³ unabhängig voneinander Wasserstoff, Halogen, Cyano, Nitro oder Amino, optional substituiert mit ein oder mehreren C₁₋₄-Alkylgruppen;
Trifluoromethyl, C₁₋₄-Alkyl, C₁₋₄-Alkoxy, -C(=O)-NH₂, C₁₋₄-Alkoxycarbonyl, Trifluoromethoxy oder Hydroxy darstellen, um (S)-3-(N-Methyl-N-benzylamino)-1-(2-thienyl)-1-propanol der Formel (IV) zu ergeben, das in Form seines Oxalatsalzes gereinigt wird, und
falls gewünscht, wird die erhaltene Verbindung der Formel (IV) gefällt in Form eines Säureadditionssalzes davon.

5. Maleatsalz des (S)-N-Methyl-N-benzyl-3-(1-naphthyloxy)-3-(2-thienyl)propanamin.

6. Verfahren zur Herstellung von (S)-N-Methyl-N-benzyl-3-(1-naphthyloxy)-3-(2-thienyl)propanamin der Formel (V) 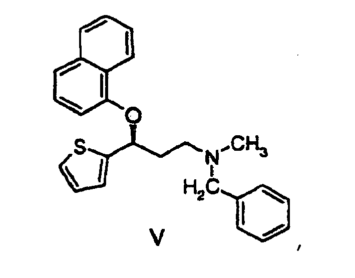 und Säureadditionssalzen davon, **dadurch gekennzeichnet, dass** 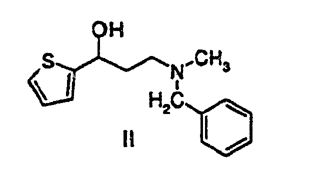 razemisches 3-(N-Methyl-N-benzylamino)-1-(2-thienyl)-1-propanol der Formel (II) mit einem D-Phenylglycinderivat der allgemeinen Formel (III) aufgespalten wird 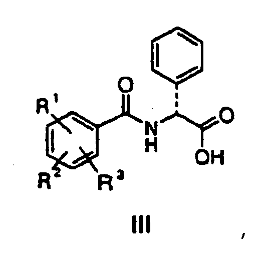 worin R¹, R² und R³ unabhängig voneinander Wasserstoff, Halogen, Cyano, Nitro oder Amino, optional substituiert mit ein oder mehreren C₁₋₄-Alkylgruppen; Trifluoromethyl, C₁₋₄-Alkyl, C₁₋₄-Alkoxy, -C(=O)-NH₂, C₁₋₄-Alkoxycarbonyl, Trifluoromethoxy oder Hydroxy darstellen, und das erhaltene (S)-3-(N-Methyl-N-benzylamino)-1-(2-thienyl)-1-propanol der Formel (IV) 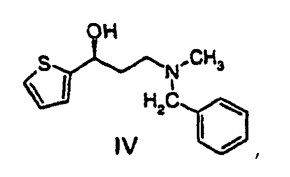 wird in Form seines Oxalatsalzes gereinigt, und
wird mit 1-Fluoronaphthalin umgesetzt, um (S)-N-Methyl-N-benzyl-3-(1-naphthyloxy)-3-(2-thienyl)propanamin der Formel (V) zu ergeben, das in Form seines Oxalatsalzes gereinigt wird, und
falls gewünscht, wird die erhaltene Verbindung der Formel (V) gefällt in Form eines Säureadditionssalzes davon.

7. Verfahren gemäß Anspruch 4 oder Anspruch 6, **dadurch gekennzeichnet, dass** die Aufspaltung mit N-Benzoyl-D-phenylglycin durchgeführt wird.

8. Verfahren gemäß irgendeinem der Ansprüche 1 und 2, **dadurch gekennzeichnet, dass** das Duloxetin ((S)-(+)-N-Methyl-3-(1-naphthyloxy)-3-(2-thienyl)propanamin) der Formel (I) durch fraktionierte Kristallisation gereinigt wird.

9. Verfahren gemäß Anspruch 8, **dadurch gekennzeichnet, dass** das Duloxetin ((S)-(+)-N-Methyl-3-(1-naphthyloxy)-3-(2-thienyl)propanamin) der Formel (I) in Form seines Maleatsalzes gefällt wird und dann das erhaltene Maleatsalz zu Duloxetin ((S)-(+)-N-Methyl-3-(1-naphthyloxy)-3-(2-thienyl)propanamin) der Formel (I) umgewandelt wird.

10. Verfahren gemäß irgendeinem der Ansprüche 1 und 2,
**dadurch gekennzeichnet, dass** das Duloxetin ((S)-(+)-N-Methyl-3-(1-naphthyloxy)-3-(2-thienyl)propanamin) der Formel (I) in Form seines Maleatsalzes gefällt wird und dann nach dem Auflösen des erhaltenen Maleatsalzes die Base freigesetzt und ohne Isolation mit wasserfreiem Chlorwasserstoff umgesetzt wird, um den Wirkstoff Duloxetinhydrochlorid zu ergeben.

11. Verfahren gemäß irgendeinem der Ansprüche 1 und 2,
**dadurch gekennzeichnet, dass** das ((S)-(+)-N-Methyl-3-(1-naphthyloxy)-3-(2-thienyl)propanamin) der Formel (I) in Form seines Maleatsalzes isoliert wird und dann ohne Freisetzung der Base das erhaltene Maleatsalz mit konzentrierter wässriger Chlorwasserstoffsäure in einem organischen Lösungsmittel umgesetzt wird, um den Wirkstoff Duloxetinhydrochlorid zu ergeben.

12. Verfahren gemäß irgendeinem der Ansprüche 1, 2, 4, 6 und 7, **dadurch gekennzeichnet, dass** das (S)-3-(N-Methyl-benzylamino)-1-(2-thienyl)-1-propanol der Formel (IV) durch fraktionierte Kristallisation gereinigt wird.

13. Verfahren gemäß Anspruch 12, **dadurch gekennzeichnet, dass** das (S)-3-(N-Methyl-benzylamino)-1-(2-thienyl)-1-propanol der Formel (IV) in Form seines Oxalatsalzes gefällt wird, und dann das erhaltene Oxalatsalz zu (S)-3-(N-Methyl-benzylamino)-1-(2-thienyl)-1-propanol der Formel (IV) umgewandelt wird.

14. Verfahren gemäß irgendeinem der Ansprüche 1, 2 und 6, **dadurch gekennzeichnet, dass** das (S)-N-Methyl-N-benzyl-3-(1-naphthyloxy)-3-(2-thienyl)propanamin der Formel (V) durch fraktionierte Kristallisation gereinigt wird.

15. Verfahren gemäß Anspruch 14, **dadurch gekennzeichnet, dass** das (S)-N-Methyl-N-benzyl-3-(1-naphthyloxy)-3-(2-thienyl)propanamin der Formel (V) in Form seines Maleatsalzes gefällt wird und dann das erhaltene Maleatsalz zu (S)-N-Methyl-N-benzyl-3-(1-naphthyloxy)-3-(2-thienyl)propanamin der Formel (V) umgewandelt wird.

## Revendications

1. Procédé pour la préparation de duloxétine 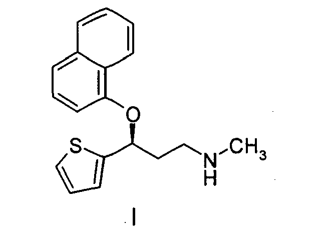 ((S)-N-méthyl-3-(1-naphtalényloxy)-3-(2-thiényl)-propanamine) de formule (I) et de ses sels pharmaceutiquement acceptables, ledit procédé comprenant les étapes suivantes :
le dédoublement du 3-(N-méthyl-N-benzylamino)-1-(2-thiényl)-1-propanol racémique de formule (II) 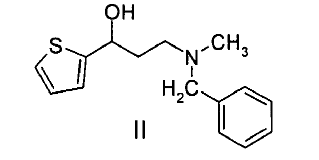
avec un dérivé de D-phénylglycine de formule générale (III) 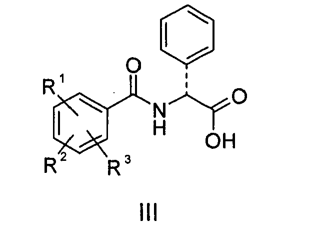
dans laquelle R¹, R² et R³ représentent, indépendamment les uns des autres, l'hydrogène, un halogène, cyano, nitro ou amino éventuellement substitué par un ou plusieurs groupes alkyle en C₁ à C₄ ; trifluorométhyle, alkyle en C₁ à C₄, alcoxy en C₁ à C₄, -C(=O) -NH₂, (alcoxy en C₁ à C₄)carbonyle, trifluorométhoxy ou hydroxy,
pour donner du (S)-3-(N-méthyl-N-benzylamino)-1-(2-thiényl)-1-propanol de formule (IV) 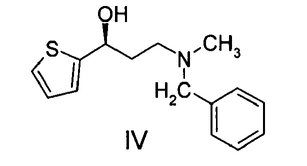
la purification du composé de formule (IV) sous la forme de son sel oxalate
la réaction du composé de formule (IV) obtenu avec du 1-fluoronaphtalène pour donner de la (S)-N-méthyl-N-benzyl-3-(1-naphtalényloxy)-3-(2-thiényl)propanamine de formule (V) 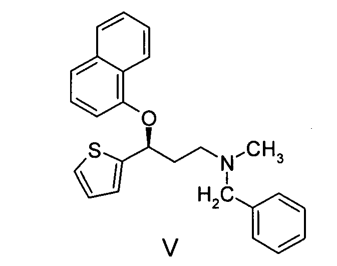
la purification du composé de formule (V) sous la forme de son sel maléate
la réaction du composé de formule (V) obtenu avec du chloroformiate de 1-chloroéthyle pour donner de la duloxétine de formule (I), et
si on le souhaite, la conversion de la duloxétine de formule (I) obtenue en un sel pharmaceutiquement acceptable de celle-ci.

2. Procédé selon la revendication 1, **caractérisé en ce que** le dédoublement est effectué avec de la N-benzoyl-D-phénylglycine.

3. Sel oxalate de (S)-3-(N-méthyl-N-benzylamino)-1-(2-thiényl)-1-propanol.

4. Procédé pour la préparation du (S)-3-(N-méthyl-N-benzylamino)-1-(2-thiényl)-1-propanol de formule (IV) 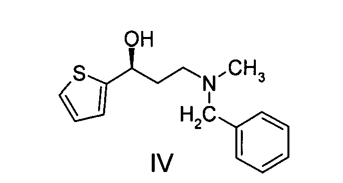 et de ses sels d'addition d'acide, **caractérisé en ce que** du 3-(N-méthyl-N-benzylamino)-1-(2-thiényl)-1-propanol racémique de formule (II) 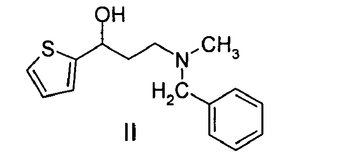 est dédoublé avec un dérivé de D-phénylglycine de formule générale (III) 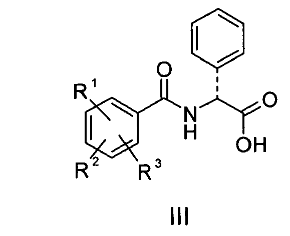 dans laquelle R¹, R² et R³ représentent, indépendamment les uns des autres, l'hydrogène, un halogène, cyano, nitro ou amino éventuellement substitué par un ou plusieurs groupes alkyle en C₁ à C₄ ; trifluorométhyle, alkyle en C₁ à C₄, alcoxy en C₁ à C₄, -C(=O)-NH₂, (alcoxy en C₁ à C₄)carbonyle, trifluorométhoxy ou hydroxy,
pour donner du (S)-3-(N-méthyl-N-benzylamino)-1-(2-thiényl)-1-propanol de formule (IV), qui est purifié sous la forme de son sel d'oxalate, et
si on le souhaite, le composé de formule (IV) obtenu est précipité sous la forme d'un sel d'addition d'acide de celui-ci.

5. Sel maléate de la (S)-N-méthyl-N-benzyl-3-(1-naphtalényloxy)-3-(2-thiényl)propanamine.

6. Procédé pour la préparation de (S)-N-méthyl-N-benzyl-3-(1-naphtalényloxy)-3-(2-thiényl)propanamine de formule (V) 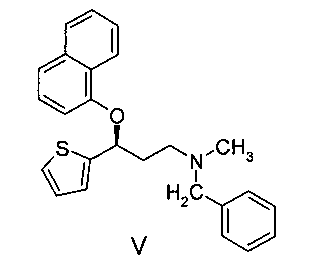 et de ses sels d'addition d'acide, **caractérisé en ce que** 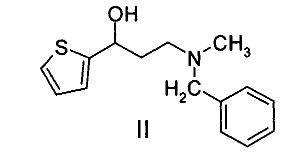 le 3-(N-méthyl-N-benzylamino)-1-(2-thiényl)-1-propanol racémique de formule (II) est dédoublé avec un dérivé de D-phénylglycine de formule générale (III) 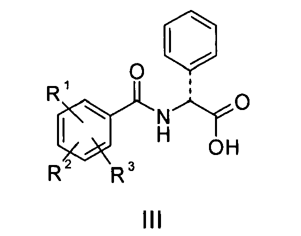 dans laquelle R¹, R² et R³ représentent, indépendamment les uns des autres, l'hydrogène, un halogène, cyano, nitro ou amino éventuellement substitué par un ou plusieurs groupes alkyle en C₁ à C₄ ; trifluorométhyle, alkyle en C₁ à C₄, alcoxy en C₁ à C₄, -C(=O)-NH₂, (alcoxy en C₁ à C₄)carbonyle, trifluorométhoxy ou hydroxy,
et le (S)-3-(N-méthyl-N-benzyl-amino)-1-(2-thiényl)-1-propanol de formule (IV) obtenu 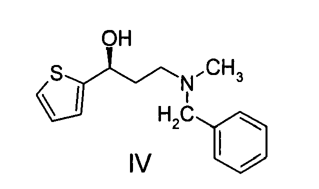 est purifié sous la forme de son sel oxalate,
et est mis à réagir avec du 1-fluoronaphtalène pour donner de la (S)-N-méthyl-N-benzyl-3-(1-naphtalényloxy)-3-(2-thiényl)propanamine de formule (V), qui est purifiée sous la forme de son sel oxalate, et
si on le souhaite, le composé de formule (V) obtenu est précipité sous la forme d'un sel d'addition d'acide de celui-ci.

7. Procédé selon la revendication 4 ou 6, **caractérisé en ce que** le dédoublement est effectué avec de la N-benzoyl-D-phénylglycine.

8. Procédé selon l'une quelconque parmi la revendication 1 et la revendication 2, **caractérisé en ce que** la duloxétine ((S)-(+)-N-méthyl-(3-(1-naphtalényloxy)-3-(2-thiényl)propanamine) de formule (I) est purifiée par cristallisation avec fractionnement.

9. Procédé selon la revendication 8, **caractérisé en ce que** la duloxétine ((S)-(+)-N-méthyl-(3-(1-naphtalényloxy)-3-(2-thiényl)propanamine) de formule (I) est précipitée sous la forme de son sel maléate, puis le sel maléate obtenu est converti en duloxétine ((S)-(+)-N-méthyl-(3-(1-naphtalényloxy)-3-(2-thiényl)propanamine) de formule (I).

10. Procédé selon l'une quelconque parmi la revendication 1 et la revendication 2, **caractérisé en ce que** la duloxétine ((S)-(+)-N-méthyl-(3-(1-naphtalényloxy)-3-(2-thiényl)propanamine) de formule (I) est précipitée sous la forme de son sel maléate puis, après dissolution du sel maléate obtenu, la base est libérée et mise à réagir sans isolation avec du chlorure d'hydrogène anhydre pour donner l'agent actif chlorhydrate de duloxétine.

11. Procédé selon l'une quelconque parmi la revendication 1 et la revendication 2, **caractérisé en ce que** la (S)-(+)-N-méthyl-(3-(1-naphtalényloxy)-3-(2-thiényl)-propanamine de formule (I) est isolée sous la forme de son sel maléate puis, sans libération de la base, le sel maléate obtenu est mis à réagir avec une solution aqueuse concentrée d'acide chlorhydrique dans un solvant organique pour donner l'agent actif chlorhydrate de duloxétine.

12. Procédé selon l'une quelconque des revendications 1, 2, 4, 6 et 7, **caractérisé en ce que** le (S)-3-(N-méthyl-N-benzylamino)-1-(2-thiényl)-1-propanol de formule (IV) est purifié par cristallisation avec fractionnement.

13. Procédé selon la revendication 12, **caractérisé en ce que** le (S)-3-(N-méthyl-N-benzylamino)-1-(2-thiényl)-1-propanol de formule (IV) est précipité sous la forme de son sel oxalate, puis le sel oxalate obtenu est converti en (S)-3-(N-méthyl-N-benzylamino)-1-(2-thiényl)-1-propanol de formule (IV).

14. Procédé selon l'une quelconque des revendications 1, 2 et 6, **caractérisé en ce que** la (S)-N-méthyl-N-benzyl-3-(1-naphtalényloxy)-3-(2-thiényl)-propanamine de formule (V) est purifiée par cristallisation avec fractionnement.

15. Procédé selon la revendication 14, **caractérisé en ce que** la (S)-N-méthyl-N-benzyl-3-(1-naphtalényloxy)-3-(2-thiényl)propanamine de formule (V) est précipitée sous la forme de son sel maléate, puis le sel maléate obtenu est converti en (S)-N-méthyl-N-benzyl-3-(1-naphtalényloxy)-3-(2-thiényl)propanamine de formule (V).
